# EUROPEAN PATENT APPLICATION

(11) **EP 3 431 996 A1**
(43) Date of publication of application: **23.01.2019**
(21) Application number: 17181657.2
(22) Date of filing: 17.07.2017
(51) Int. Cl.: G01N 33/573, G01N 33/86

(54) **QUANTIFYING ENZYMATIC ENTITIES AND THEIR ACTIVE SITES**

(71) Applicant: PreviPharma Consulting GmbH, 68165 Mannheim (DE)
(72) Inventor: Mandago, Maurice, 69250 Schönau (DE); Gruber, Ricarda, 69256 Mauer (DE); Gerding, Hanne Rieke, 68229 Mannheim (DE); Kießig, Stephan T., 69168 Wiesloch (DE)
(74) Representative: Jacobi, Markus Alexander

(57) **Abstract**

The present invention relates to a method for determining the content of an enzymatic entity E and the content and accessibility of one or more active sites of entity E in a liquid L comprising the steps of contacting L with a first binding moiety A1 that binds to a first binding site B1 of E that is different from the ligand binding site B2, and with a second binding moiety A2 that binds to B2. Furthermore, the present invention refers to a composition and a kit for use in the context of such method.

## Description

The present invention relates to a method for determining the content of an enzymatic entity E and the content and accessibility of one or more active sites of entity E in a liquid L comprising the steps of contacting liquid L with a first binding moiety A1 that binds to a first binding site B1 of entity E that is different from the ligand binding site B2, and with a second binding moiety A2 that binds to B2. Furthermore, the present invention refers to a composition and a kit for use in the context of such method.

A large variety of pathologic conditions are known which are associated with altered levels and/or activities of enzymatic entities. Such pathologic condition can be caused by a decreased or an enhanced level (content) of the enzymatic entity in a body fluid (e.g., blood). Alternatively or additionally, it can also be caused by a decreased or an enhanced level of the enzymatic activity in a body fluid (e.g., blood). In other words, even if the overall content of enzymatic entities remains essentially unchanged, the amount of active sites that are suitable for binding the enzyme's ligand may be decreased. Both, the overall enzyme content as well as its the content and accessibility of active sites of the enzyme may have an impact on enzymatic activity.

As one example, hemophilia A (bleeding disorder) is mainly caused by a missing coagulation factor VIII activity, leading into a dysregulation of the enzymatic cascade associated with blood coagulation. In a first step, it is of interest to determine which coagulation factor is associated with the individual's hemophilia. But even if the coagulation factor associated with the individual's hemophilia is known, individual's hemophilia can have two reasons: i) altered blood levels of the respective coagulation factor and/or ii) altered activity of the respective coagulation factor due to altered content and/or accessibility of the active site of the coagulation factor. The effect on such patient when administering the respective coagulation factor may be different upon the reasoning of the patient's hemophilia. When the coagulation factor is generally present in the patient's blood, but merely the active sites are not available, the patient's immune system will likely be familiar with the presence of the coagulation factor and most likely no immune response will occur.

When, in contrast, the coagulation factor is inherently absent in the patient's body, there is a considerable likelihood that the patient's body might bear undesired immune response to the administered coagulation factor considered as foreign by the patient's immune system.

A well-known gene defect is e.g. a defect of factor VIII which is also described as anti-hemophilic factor (AHF). Factor VIII is a cofactor for factor IXa (activated from of antihemophilic factor B, Christmas factor, EC 3.4.21.22) which, in the presence of calcium ions and phospholipids, forms a complex enzymatic entity that enables convertion of factor X (also: Stuart-Prower factor, EC 3.4.21.6) into its activated form Xa. In one group of patients, the total content of factor VIII is diminished. In another group of patients, factor VIII is altered in structure and the complex enzymatic entity of factors VIII and IX does not form an active site. A patient's therapy depends on whether factor VIII is absent or an inactive form of factor VIII is present in the patient's blood. When factor VIII is absent in the patient, there is a considerable risk of undesired immune reactions when supplying the patient with factor VIII supplements, whereas there is merely a far lower risk of such immune reaction when the patient bears an altered form of factor VIII. Accordingly, it is of interest to obtain information on the content and, concomitantly on the presence of active sites and thus the activity of the factor VIII present in the patient's blood.

Further, some coagulation factors such as, e.g., plasminogen, are present in various isotypes in a healthy individual. Also here, total contents as well as contents of active sites and optionally also information on the levels of the different isotype are of interest.

Contents of enzymatic entities in liquids are routinely determined by a number of quantitative methods such as, e.g., enzyme-linked immunosorbent assay (ELISA) (cf., pages 181-183 of the textbook "Molekulare Biotechnologie - Grundlagen und Anwendungen", 2009, ed. Clark and Pazdernik, Spectrum Akademischer Verlag Heidelberg). An ELISA method bases on a binding moiety that specifically binds to the enzymatic entity of interest which is immobilized on a solid phase. Then, the liquid containing the enzymatic entity of interest is contacted with the immobilized binding moiety and is thereby immobilizing the enzymatic entity of interest on the solid phase. Typically, in a subsequent step, the liquid is removed, optionally followed by a washing step, and a second binding moiety which also binds to the enzymatic entity of interest is contacted with the immobilized enzymatic entity of interest.

This second binding moiety often bears a detectable moiety enabling the determination of the content of the enzymatic entity of interest, often called "titer" (an equivalent for the concentration defined by the last detectable dilution of a sample) of the enzymatic entity of interest. A significant drawback of such method like ELISA is that it merely bears information on the content of the enzymatic entity of interest, but no indication of the content and accessibility of its active sites and, thereby of its activity. Detection merely depends on the presence of certain epitopes, but it is independent of an accessible active site of the enzymatic entity of interest.

On the other hand, there are methods such as activity assays (often based on enzyme kinetics) which focus on the activity level only. Here, typically, the catalytic turnover rate of an analyte of interest is determined, often by means of using a labelled or chromogenic substrate. Such activity levels bear the significant drawback that the total content of enzymatic entities is not determined and that it is often not clear which of the enzymatic entities that may be comprised in the liquid are responsible for the catalytic activity. Further, in such activity assay, catalytically inactive forms (pro-enzymes, degraded enzymes, enzyme-inhibitor complexes) are typically completely disregarded - although still bearing biological effects such as, e.g., immunogenicity.

Therefore, today's methods typically provide either information on the total content of enzymatic entities or on the presence of active sites of the enzymatic entities. If information on both is desired, mostly two separate or highly complex and laborious tests are needed. Accordingly, there is an unmet need for less laborious methods that concomitantly provide information on the total content of an enzymatic entity and on the content and accessibility of its active sites in a liquid of interest.

Surprisingly, it has been found that the content of an enzymatic entity and the content and accessibility of its active sites can both be obtained by a method based on the determination of detectable binding moieties specifically binding to the enzymatic entity of interest and, concomitantly, of detectable binding moieties specifically binding its accessible active site. This method is based on a solid phase and can also be performed easily on side.

A first aspect of the invention relates to a method for (concomitantly) determining the content of an enzymatic entity E and the content and accessibility of one or more active site(s) of E in a liquid L, said method comprising the following steps:
(i) providing the following:
   (A1) a first binding moiety A1 that binds to a first binding site B1 of E that is different from the ligand binding site B2 in the active site of E, wherein A1 bears a first detectable moiety M-I,
   (A2) a second binding moiety A2 that binds to B2, and
   (A3) optionally a third binding moiety A3 that binds to a third binding site B3 of E that is different from B1 and B2,
   wherein a single one of A3 or A2 is bound to a solid phase S, and wherein, if A3 is present, the other one of A2 or A3 not bound to the solid phase S bears a second detectable moiety M-II that is different from M-I;
(ii) contacting L comprising E with A1 and A2 and, if present, A3 and enabling binding of A1 and A2 and, if present, A3 to E;
(iii) removing unbound A1 and A2 and, if present, A3 from the solid phase S; and
(iv) determining the amount of M-I and, if present, M-II immobilized on the solid phase S.

The method of the present invention is for a (concomitantly) quantification of the content of an enzymatic entity E and the content and accessibility of the one or more active site(s) of said enzymatic entity E in a liquid L, said method comprising the above steps (i)-(iv). This method enables the concomitant determination of the content of an enzymatic entity E and the content and accessibility of the active sites of the enzymatic entity E in a liquid L.

The method of the present invention is usable in any context where it is desired to obtain information of the quantity of the content of an enzymatic entity E and the content and accessibility of one or more active site(s) of E in a liquid L. The method may be used in scientific research, may be used in a medicinal/clinical context (e.g., as a point of care (PoC) test), may be used for home care purposes, may be conducted to control a production process and/or for other analytical purposes. It may be conducted just on side (also designated as a point of care (PoC) test, e.g., bedside, at home or the like) or may be conducted in a laboratory.

The method may be conducted as or may be combined with an enzyme-linked immunosorbent assay (ELISA), a complement-enzyme linked immunosorbent assay (CELISA), a line assay, a dot assay and an agglutination assay. It may provide qualitative, quantitative or semi-quantitative data.

Preferably, the step (i) is performed as a first step. Then, preferably, as a subsequent step (ii) of contacting L with at least one of A1 and/or A2 and/or, if present, A3 and/or, if present, A4 is performed. Then, preferably, as a subsequent third step (iii) following step (ii) is performed, said step (iii) comprising removing the amount of the respective unbound binding moiety/moieties. In a forth step (iv) following preceding step (iii), the respective binding moiety/moieties may be determined.

As used in the context of the present invention, the term "enzymatic entity" may be understood in the broadest sense as a molecular unit of interest that consists of or comprises an enzyme or inactive homologue thereof. An enzymatic entity E may be an enzyme (or inactive homologue thereof), or may be a complex comprising an enzyme (or inactive homologue thereof) and one or more other further molecular structures that may exemplarily be cofactors and/or regulators thereof. In a preferred embodiment, such further molecular structure is a cofactor which is a polypeptide. Each of the binding moieties (A1, A2, A3 and A4) may specifically bind to the enzyme or may bind to a further molecular structure such as a cofactor and/or a regulator comprised in the enzymatic entity E. The enzymatic entity E may be catalytically active or may be catalytically inactive. In a particularly preferred embodiment, the enzymatic entity E is an enzyme.

As used in the context of the present invention, the term "enzyme" may be understood in the broadest sense as understood by the person skilled in the art. Thus, an enzyme may generally be any macromolecular biological catalysts that enables catalyzing the conversion of the at least one substrate into the respective product. Typically, an enzyme known in the art is designated by a widely accepted enzyme classification (EC) number.

Typically and particularly preferably but not necessarily an enzyme in the context of the present invention is or is mainly composed of a polypeptide. Alternatively, the enzyme may be or may be mainly composed of a nucleotide (e.g., ribonucleic acid (RNA)).

As use herein, the term "active site" may be understood in the broadest sense as the structural region of the enzymatic entity E where the one or more substrates bind and may undergo a chemical reaction. Typically, the active site comprises moieties (mostly amino acid moieties) that are able to form temporary bonds with the one or more substrates (i.e., the substrate binding site) and moieties (mostly amino acid moieties) that enable catalyzing a reaction of that one or more substrates to the respective one or more products (and vice versa) (i.e., the catalytic site).

Preferably, the active site is a groove or pocket of the enzymatic entity E which may optionally be located in a tunnel within the enzymatic entity E or may be located between the interfaces of multimeric enzymatic subunits of the enzymatic entity E. The content of an active site in the liquid L herein describes how many active site units are present in L. For ligand binding of the respective ligand of the enzymatic entity E, not only the quantity of the active sites is of interest, but likewise its accessibility.

As used in the present invention, the term "accessibility of one or more active sites of E" may be understood in the broadest sense as enabling binding of the ligand of the enzymatic entity E to the active site B2 of E. In other words, when the binding site is accessible, the binding moiety A2 can bind to its binding site B2. When the binding moiety A2 cannot bind to its binding site B2, the active site of E is not accessible. This may exemplarily be due to an alteration of the structure of E (e.g., by means of truncation, elongation or the like, or rearrangement of subunits) or may be due to the presence of competing ligands or ligand analogues present in the enzymatic entity E and/or the liquid L of interest. Preferably, the active site of which its content and accessibility is determined is considerable (essentially) equal to the binding site B2 therein (i.e., preferably, but not necessarily: active site = B2)

In the context of the present invention, the terms "polypeptide" and "protein" may be understood interchangeably in the broadest sense as a compound mainly composed of natural amino acid moieties consecutively conjugated with another via amide bonds. A polypeptide in the sense of the present invention may or may not be subjected to one or more posttranslational modification(s) and/or be conjugated with one or more non-amino acid moiety/moieties. The termini of the polypeptide may, optionally, be capped by any means known in the art, such as, e.g., amidation, acetylation, methylation and acylation.

Posttranslational modifications are well-known in the art and may be but may not be limited to lipidation, phosphorylation, sulfatation, glycosylation, truncation, oxidation, reduction, decarboxylation, acetylation, amidation, deamidation, disulfide bond formation, amino acid addition, cofactor addition (e.g., biotinylation, heme addition, eicosanoid addition, steroid addition) and complexation of metal ions, non-metal ions, peptides or small molecules and addition of iron-sulphide clusters. Moreover, optionally, co-factors, in particular cyclic guanidinium monophosphate (cGMP), but optionally also such as, e.g., ATP, ADP, NAD⁺, NADH+H⁺, NADP⁺, NADPH+H⁺, metal ions, anions, lipids, etc. may be bound to the polypeptide, irrespective on the biological influence of these co-factors.

Preferably, at least the method steps (ii) and (iii) are each independently from another conducted in a liquid such as, e.g., a buffer suitable for dissolving the respective binding moieties and maintain the structural integrity of the enzymatic entity E. More preferably, all of the method steps (i), (ii), (iii) and (iv) are each independently from another conducted in a liquid such as, e.g., a buffer suitable for dissolving the respective binding moieties and maintain the structural integrity of the enzymatic entity E.

As use herein, the term "specifically bind to" may be understood as binding to its binding site (i.e., the respective target structure) with a higher affinity than to other molecular structures. Preferably, the respective binding moiety binds to its molecular target (e.g., A1 to B1, A2 to B2, A3 to B3, A4 to B4) with an affinity that is at least 10fold, more preferably at least 100fold, even more preferably at least 1000fold, higher than to other molecular structures. Exemplarily, A1 binds to B1 with an activity that is at least 10fold, more preferably at least 100fold, even more preferably at least 1000fold, higher than to B2, B3 and B4. *Mutatis mutandis,* also A2, A3 and A4 bind to their respective targets with such higher affinity as indicated before.

As used in the context of the binding moieties, a "binding site" may be understood in the broadest sense as a region of the enzymatic entity E to which the respective binding moiety can bind to. When the binding moiety is an antibody or a fragment or analogue thereof, the binding site may also be designated as "epitope". The binding site may be a linear binding site depending on a section of the consecutive polypeptide strand of E, may be a structural binding site depending on the structural arrangement of amino acid moieties in the structure of E, may also depend on non-amino acid moieties, or may be a mixture of two or more thereof.

In order to improve comparability of the contents determined in different liquids L with another, in other words to normalize the determined values, the determined contents of an enzymatic entity E and the content and accessibility of one or more active site(s) of E in a liquid L may optionally be compared to respective reference values. Such reference values may be each an internal control (i.e., a further control sample measured under comparable conditions, preferably in the same test series) with known contents of enzymatic entity E and the content and accessibility of one or more active site(s) of E, or may be each a predetermined reference value typically but not necessarily obtained from one or more previous measurements conducted under comparable conditions wherein the respective content of enzymatic entity E and active sites, respectively, is known.

Accordingly, in a preferred embodiment, the method further comprises the step of comparing the determined content of enzymatic entity E and/or content of active sites with
(a) a predetermined reference value indicating the dependency of respective signal intensity arising from the respective detectable moiety (i.e., M-I, M-II or M-III) to the respective content (i.e., content of enzymatic entity E and/or content of active sites), or
(b) the respective content with a control series of various contents in control samples measured under comparable conditions, preferably in the same measurement series.

Accordingly, when A3 is immobilized to the solid phase S, in a preferred embodiment, the method further comprises the step of comparing the determined content of enzymatic entity E with
(a) a predetermined reference value indicating the dependency of signal intensity arising from M-I to the content of enzymatic entity E, or
(b) the content of enzymatic entity E with of the signal intensity arising from M-I in a control series of various contents of enzymatic entity E in control samples measured under comparable conditions, preferably in the same measurement series.

Accordingly, when A3 is immobilized to the solid phase S, in a preferred embodiment, the method further comprises the step of comparing the determined content of active sites of enzymatic entity E with
(a) a predetermined reference value indicating the dependency of signal intensity arising from M-II to the content of active sites of enzymatic entity E, or
(b) the content of active sites of enzymatic entity E with of the signal intensity arising from M-II in a control series of various contents of active sites of enzymatic entity E in control samples measured under comparable conditions, preferably in the same measurement series.

Accordingly, when A2 is immobilized to the solid phase S, in a preferred embodiment, the method further comprises the step of
comparing the determined content of active sites of enzymatic entity E with
(a) a predetermined reference value indicating the dependency of signal intensity arising from M-I to the content of active sites of enzymatic entity E, or
(b) the content of active sites of enzymatic entity E with of the signal intensity arising from M-I in a control series of various contents of active sites of enzymatic entity E in control samples measured under comparable conditions, preferably in the same measurement series.

When the relationship between the signal intensity arising from a control sample and the respective content (e.g., content of enzymatic entity E or content of active sites of E) is known, the method may be used for determining the (absolute) quantity of the respective moiety of interest (e.g., enzymatic entity E or active sites of E). Therefore, a preferred embodiment of the present invention relates to the method as laid out herein for determining the quantity of the content of an enzymatic entity E and/or its active sites of interest in a liquid L.

As used in the context of the present invention, the term "content" may be understood in the broadest sense as quantity of the determined component of interest that is present in the liquid L. When the content of an enzymatic entity E is determined, E present in the liquid L is quantified. When the content and accessibility of one or more active site(s) of E is determined, active sites that are present in the liquid L are quantified.

These quantified contents may be normalized or relative contents. Normalized contents may enable the comparability between different samples.

This typically presupposes that the measurement conditions (i.e., comparable volume of the liquid L used in combination with a solid phase S and binding moieties (A1, A2, optionally A3 and optionally A4) each having comparable properties) are used. When referred to a known relationship between the signal intensity arising from the respective detectable moiety (M-I, M-II or M-III) in step (iv) and the concentration of the respective component, normalized contents may also indicate the concentration (e.g., in nM/ml or µM/ml). When referred to a known relationship between the signal intensity arising from the respective detectable moiety (M-I, M-II or M-III) in step (iv) and the total amount of the respective component, preferably normalized contents may also indicate a total (molar) amount (e.g., in pmol or nmol) comprised in the liquid L employed in the method. It will be understood that when knowing the volume of the liquid L employed in the method, the total (molar) amount can be easily be mathematically converted into the concentration and vice versa.

Alternatively, the term "content" may also be the relative content. The relative content may be just comparing the relative signal intensities arising from the respective detectable moiety (M-I, M-II or M-III) in step (iv) with another. Comparing these values with one or more control samples may provide relative conclusions such as, e.g., "higher than control" or "lower than control". When the control sample exemplarily is a healthy individual, it may be determined how the relative content determined is in relation to the healthy individual. When it is known with deviation from the healthy individual indicates a pathological condition, the method may also provide information on the individual's health status.

In a preferred embodiment, the term "content" as used herein reflects the concentration or the total amount of the determined component that is present in the liquid L.

In a more preferred embodiment, the term "content" as used herein reflects the concentration of the determined component that is present in the liquid L. Then, in the wording as used herein the term "content" may also be replaced by the term "concentration". In other words, when the quantity of the content of an enzymatic entity E and the content and accessibility of one or more active site(s) of E is normalized to a certain volume used, in particular when in the control and test samples the same volume is used, the concentration (e.g., in nM/ml or µM/ml) of an enzymatic entity E and/or the concentration (e.g., in nM/ml or µM/ml) of the accessible active sites of E may be determined.

Therefore, a preferred embodiment of the present invention relates to the method as laid out herein for determining the concentration of the content of an enzymatic entity E and/or its accessible active sites of interest in a liquid L.

As used in the context of the present invention, the term "solid phase S" may be understood in the broadest sense as any carrier based on a solid phase that is suitable for binding to a binding moiety A2 or A3. Herein a solid phase may be understood in the broadest sense as generally understood by a person skilled in the art, i.e., as any material that can bear a given form at ambient temperature (20°C), which is thus not liquid, fluid, pasty or gaseous. A solid phase may also be a solid support. It will be understood that the solid phase S as used in the context of the present invention will typically show no or no significant unspecific binding to E. In other words, at least 75% by weight, preferably at least 90% by weight, more preferably at least 95% by weight, in particular at least 99% by weight, of E immobilized on the solid phase E is immobilized via A2 or A3 (and thus not via unspecific adhesion to the solid phase's surface). The solid phase S may optionally be hard, dimensionally stable, wax-like, elastic, flexible, thermoelastic and/or kneadable. The binding to a binding moiety A2 or A3 may occur on the surface of the solid phase S and/or in its interior, in particular in parts of the solid phase S that are also accessible to the enzymatic entity E. This means that in particular in a porous or gel-like solid phase S, binding moiety A2 or A3 may optionally also be bound in the interior of the solid phase S.

Exemplarily, the solid phase S may be selected from the group consisting of a reaction vessel or part thereof (e.g., a multiwell plate, in particular the bottom thereof), a bead (e.g., a micro- or nanobead), a microarray, a film (e.g., a polymer film), a tissue (e.g., a cellulose, a nitrocellulose), a sponge, and a gel (e.g., a hydrogel). Exemplarily, the solid phase S may comprise or (essentially) consist of organic polymers (e.g., thermoplastic moldings (e.g., polypropylene (PP), polyvinylchloride (PVC), polystyrene (PS), or mixtures of two or more thereof), (bio)ceramics (e.g., hydroxyapatite, (tri-)calcium phosphate, or mixtures thereof), polysaccharides or derivatives thereof (e.g. cellulose and derivatives thereof (e.g., cellulose acetate or nitrocellulose, tissue, or (blotting) paper), agarose, Sepharose, Sephadex or mixtures of two or more thereof), glass (typically mostly composed of silicon dioxide), aluminum, a gelling polypeptide (e.g., gelatin), and combinations of two or more thereof. Examples for (bio)ceramics usable in the context of the present invention are provided in Wang et al. (Interface Focus (2012) 2:259-277).

The person skilled in the art knows that, when the surface is reactive with polypeptides such as E and/or any of A1-A4, after binding the desired binding moiety A2 or A3, the remaining reactive groups of the solid phase S are preferably inactivated prior to conducting the method of the present invention. Exemplarily, depending on the reactive groups used, this may be conducted by hydrolysis or blocking with other polypeptides (e.g., albumin).

The step (i) of providing the components may be conducted in any means. Exemplarily, the binding moieties A and A2 and, optionally, A3 and/or A4 may be obtained from commercial sources or may be prepared.

The step (ii) of contacting the liquid L comprising the enzymatic entity E with the binding moieties may be conducted in any means. Preferably, the step (ii) comprises at least two sub-steps:
(ii-a) contacting L comprising E with the binding moiety bound to the solid phase S and enabling binding of E to this binding moiety bound to the solid phase S, preferably followed by at least one washing step; and
(ii-b) contacting L comprising E (concomitantly or sequentially) with the one or more other binding moiety/moieties not bound to the solid phase S and enabling binding of E to said one more other binding moiety/moieties.

Accordingly, in the particularly preferred embodiment when A3 is bound to the solid phase S, the step (ii) comprises at least two sub-steps:
(ii-a) contacting L comprising E with A3 bound to the solid phase S and enabling binding of E to A3 bound to the solid phase S, preferably followed by at least one washing step; and
(ii-b) contacting L comprising E (concomitantly or sequentially) with A1 bearing M-I and A2 bearing M-II and, optionally, A4 bearing M-III.

Alternatively, when A2 is bound to the solid phase S, the step (ii) comprises at least two sub-steps:
(ii-a) contacting L comprising E with A2 bound to the solid phase S and enabling binding of E to A2 bound to the solid phase S, preferably followed by at least one washing step; and
(ii-b) contacting L comprising E (concomitantly or sequentially) with A1 bearing M-I and, optionally, A3 bearing M-II and, optionally, A4 bearing M-III.

As used herein, the term "enabling binding" may be understood in the broadest sense as forming any kind of bond. This may be a non-covalent or a covalent bond. Preferably, a non-covalent bond of a dissociation constant Kd of not higher than 100 nmol/L, more preferably not higher than 100 nmol/L, in particular not higher than 50 nm is formed. When a binding moiety is an antibody or an analogue or truncated from thereof, the Kd may even be lower than 20 nmol/L. Enabling binding may be achieved by contacting and incubating the binding moiety with its binding partner E at conditions suitable for forming such bond. Typically, binding may be achieved by contacting the respective binding moiety/moieties dissolved or suspended in a liquid, in particular an aqueous buffer maintains the structural integrity of E.

This is particularly beneficial when E is intended to be bound to A2, because this preferably requires the structural integrity of the active site as a binding pocket.

Optionally, a buffer as used in the context of the present invention may comprise, in addition to one or more buffer agents (e.g., selected from phosphate/bisphosphate salts, HEPES, etc.), a soluble polymer such as polyethylene glycol (PEG), a detergent (e.g., Tween, Triton, sodium dodecyl sulfate (SDS), dimethyl sulfoxide (DMSO), or combinations of two or more thereof). Optionally, the buffer may bear a pH in the range of from 5 to 8, preferably 5.5 to 8, in particular 6.5 to 7.5.

The step (iii) of removing unbound binding moieties may be conducted in any means. This step may include the step of removing a liquid comprising the binding moieties and optionally one or more washing steps. Exemplarily, after conducting step (ii) of enabling binding of the binding moieties, the remaining portions of unbound binding moieties remaining in the supernatant liquid are removed (e.g., by means of discarding of suction). This may optionally have followed by one or more washing steps (e.g., by means of a buffer maintaining the structural integrity of E and the binding moieties bound thereto, in other words, maintain the binding of E to the respective binding moieties). The step (iii) may be conducted in several sub-steps subsequent to respective sub-steps of step (ii) as exemplified above.

Preferably, the unbound binding moieties A1 and A2 and, if present, A3 do not specifically bind to E. More preferably, such binding moieties do not specifically bind to E and to the other binding moieties. Even more preferably, such binding moieties do (essentially) not bind to any solid material, in particular not to the solid phase S and to the other binding moieties (beyond weak surface interactions with solid parts). It will be understood that some weak surface interactions with solid parts might optionally occur. In summary, the unbound binding moieties A1 and A2 and, if present, A3 are preferably dissolved in a buffer and can optionally be removed by discarding, sucking or the like as described herein.

The step (iv) of determining the amount of the one or more bound binding moiety/moieties may be conducted in any means. The means for determining may be any means usable for this purpose. Examples are provided below. It will be understood that the means to perform the step (iv) will be adapted to the respective detectable moieties (M-I, M-I and/or M-III). Depending on the detectable moieties M-I, M-II and M-III, as far as present, used, various routes of detection may be employed such as, exemplarily, colorimetry, enzymometry, turbidimetry, nephelometry, (chemo)luminometry, fluorometry (including quenching- and/or FRET- and/or FRAP-based methods and time-resolved fluorometry), a scintillation assay, or combinations of two or more thereof. A number of detection steps that may be used in the context of the present invention are also summarized in Kiessig (cf., BioTec (1991) 4:30-34) and in Porstmann and Kiessig (Journal of Immunological Methods (1992) 150:5-21). The person skilled in the art will immediately know how to conduct these detection steps to obtain (semi)quantitative values. Exemplarily, colorimetry may be conducted by means of a photometer.

In a particularly preferred embodiment, the method of the present invention comprises the following steps:
(i) providing the following:
   (A1) a first binding moiety A1 that binds to a first binding site B1 of E that is different from the ligand binding site B2 in the active site of E, wherein A1 bears a first detectable moiety M-I,
   (A2) a second binding moiety A2 that binds to B2, wherein A2 bears a second detectable moiety M-II that is different from M-I, and
   (A3) a third binding moiety A3 that binds to a third binding site B3 of E that is different from B1 and B2, wherein said A3 is bound to a solid phase S, and
   (A4) optionally a forth binding moiety A4 that binds to a binding site B4 that is not present in all isotypes of E, wherein B4 is different from each of B1, B2 and B3;
(ii-a) contacting L comprising E with A3 bound to the solid phase S and enabling binding of E to A3 bound to the solid phase S;
(iii-a) removing unbound A3 from the solid phase S, preferably followed by at least one washing step;
(ii-b) contacting L comprising E (concomitantly or sequentially) with A1 bearing M-I and A2 bearing M-II and, if present, A4 bearing M-III.
(iii-b) removing unbound A1 and A2 and, if present, A4 from the solid phase S, preferably followed by at least one washing step; and
(iv) determining the amount of M-I and M-II and, if present, M-III immobilized on the solid phase S.

In an alternative preferred embodiment, the method of the present invention comprises the following steps:
(i) providing the following:
   (A1) a first binding moiety A1 that binds to a first binding site B1 of E that is different from the ligand binding site B2 in the active site of E, wherein A1 bears a first detectable moiety M-I, and
   (A2) a second binding moiety A2 that binds to B2, wherein said A2 is bound to a solid phase S, and
   (A4) optionally a forth binding moiety A4 that binds to a binding site B4 that is not present in all isotypes of E, wherein B4 is different from each of B1 and B2;
(ii-a) contacting L comprising E with A2 bound to the solid phase S and enabling binding of E to A2 bound to the solid phase S;
(iii-a) removing unbound A2 from the solid phase S, preferably followed by at least one washing step;
(ii-b) contacting L comprising E (concomitantly or sequentially) with A1 bearing M-I and, if present, A4 bearing M-III.
(iii-b) removing unbound A1 and, if present, A4 from the solid phase S, preferably followed by at least one washing step; and
(iv) determining the amount of M-I and, if present, M-III immobilized on the solid phase S.

The second binding moiety A2 may be any molecular entity that specifically binds to the ligand binding site B2 of the enzymatic entity E. The ligand binding site B2 preferably is present in the active site of E.

In a preferred embodiment, the second binding moiety A2 is a substrate analogue of E. In a more preferred embodiment, the second binding moiety A2 is a substrate analogue inhibitor of E.

Particularly preferably, the substrate analogue inhibitor of E specifically binds to the active sites. Then, the amount of bound A2 may correlate with enzymatic activity. Assuming a linear relationship between the content of active sites and enzymatic activity (i.e., a kinetic first order relationship), the content of active sites may indicate the catalytic activity. Accordingly, in a preferred embodiment, the method of the present invention is for determining the content of an enzymatic entity E and its enzymatic activity in a liquid L. When E is an enzyme, in a preferred embodiment, the method of the present invention is for determining the content of an enzyme E and its enzymatic activity in a liquid L. When the enzymatic entity E is or comprises fibrin and/or fibrinogen, the substrate analogue (inhibitor) may exemplarily be D-phenylalanyl-prolyl-arginyl chloromethyl ketone (PPACK). In the sense of the present invention, PPACK may be either labelled with a detectable moiety (e.g., a biotinyl moiety enabling binding by (strept)avidin (enzyme fusion protein)) or may be bound to the solid phase S.

The liquid L of interest may in principle be any liquid sample that comprises the enzymatic entity E. In a preferred embodiment, the liquid L is obtained from a biological source. Exemplarily, such biological source may be selected from the group consisting of a liquid sample obtained from a human, a non-human animal, a plant, a human cell or a medium its cultured in, a non-human animal cell or a medium its cultured in, a plant cell or a medium its cultured in, a bacterial cell or a medium its cultured in, and a fungal cell or a medium its cultured in. Alternatively, the liquid L may also be a processed or synthetical sample. In a more preferred embodiment, the liquid L is a body fluid or a liquid derived therefrom. More preferably, the liquid L is a body fluid or a liquid derived therefrom obtained from a human or non-human animal body. In a particularly preferred embodiment, the liquid L is blood plasm or a fraction thereof. More preferably, the liquid L is a blood plasm or a fraction thereof obtained from a human or non-human animal body.

The enzymatic entity E may be any enzymatic entity E. It may be an enzymatic entity E obtained or obtainable from a human, a non-human animal, a plant, a human cell or a medium its cultured in, a non-human animal cell or a medium its cultured in, a plant cell or a medium its cultured in, a bacterial cell or a medium its cultured in, and a fungal cell or a medium its cultured in. Alternatively, the enzymatic entity E may also be a processed or synthetical sample. In a more preferred embodiment, the enzymatic entity E is obtained or obtainable from a body fluid or a liquid derived therefrom. More preferably, the enzymatic entity E is obtained or obtainable from a body fluid or a liquid derived therefrom obtained from a human or non-human animal body. In a particularly preferred embodiment, the enzymatic entity E is obtained or obtainable from blood plasm or a fraction thereof. More preferably, the enzymatic entity E is obtained or obtainable from blood plasm or a fraction thereof obtained from a human or non-human animal body. In a particularly preferred embodiment, the enzymatic entity E is characterized in that it is a coagulation factor. Such coagulation factor may be obtained or obtainable from blood plasm or a fraction thereof obtained from a human or non-human animal body.

As used in the context of the present invention, the terms "coagulation factor" and "blood-coagulation factor" may be understood interchangeably in the broadest sense as any enzymatic entity E comprised in an individual's blood (in particular blood plasm) that may have an impact on coagulation (also known as clotting) of blood, i.e., the formation of a blood clot. Exemplarily, a coagulation factor which is an enzyme may be selected from the group consisting of factor II (also: (pro)thrombin, fibrinogenase, thrombase, tropostasin, factor IIa, enzyme classification (EC) 3.4.21.5), factor V (also: proaccelerin, labile factor), factor VII (also: proconvertin, factor VIIa, EC 3.4.21.21), factor VIII (also: anti-hemophilic factor (AHF)), factor IX (also: antihemophilic factor B, Christmas factor, EC 3.4.21.22), factor X (also: Stuart-Prower factor, EC 3.4.21.6), factor XI (plasma thromboplastin antecedent, EC 3.4.21.27), factor XII (Hageman factor, EC 3.4.21.28), prekallikrein (Fletcher factor, EC 3.4.21.34), protein C (EC 3.4.21.69), plasminogen (EC 3.4.21.7), tissue plasminogen activator (also tPA, PLAT, EC 3.4.21.68), urokinase (also: urokinase-type plasminogen activator, uPA, EC 3.4.21.73), factor XIII (fibrin-stabilizing factor, EC 2.3.2.13), and cancer procoagulant.(EC 3.4.22.26). It will be understood that in principle all isotypes, zymogens and matured forms of the aforementioned enzymes are included in the sense of the present invention.

Likewise, as laid out above, it will be understood that alternatively also any other enzymatic entities, including enzymes, enzyme complexes as well as isotypes, zymogens and matured forms thereof, are also included in the sense of the present invention.

The enzymatic entity E may have any catalytic activity and structure. In a preferred embodiment, the enzymatic entity E is or comprises a protease. As used herein, the terms "protease" and "peptidase" used without any further specification may be understood interchangeably in the broadest sense as any enzyme that enables (typically selective) cleavage of peptide bonds (also: amide bonds) between two amino acid moieties in a polypeptide sequence.

In a more preferred embodiment, the enzymatic entity E is or comprises a serine protease. In a more preferred embodiment, the enzymatic entity E is a serine protease.

In another preferred embodiment, the enzymatic entity E is or comprises a coagulation factor. More preferably, the enzymatic entity E is a coagulation factor. In more preferred embodiment, the enzymatic entity E is or comprises a coagulation factor and in that it is or comprises a protease. In an even more preferred embodiment, the enzymatic entity E is a coagulation factor and is a protease.

In a highly preferred embodiment, the enzymatic entity E is characterized in that it is or comprises a coagulation factor and in that it is or comprises a serine protease. In a particularly preferred embodiment, the enzymatic entity E is characterized in that it is a coagulation factor and in that it is a serine protease.

The term "serine protease" may be understood in the broadest sense as any enzyme that may catalyze the cleavage of a peptide bone in a polypeptide, wherein the active site of the enzyme comprises a serine moiety (as an nucleophilic amino acid moiety). Preferably, a serine protease of the present invention is a serine endopeptidase (enzyme classification (EC) 3.4.21.).

Exemplarily, a coagulation factor that is a serine protease may be selected from the group consisting of factor II (also: (pro)thrombin, fibrinogenase, thrombase, tropostasin, factor IIa, EC 3.4.21.5), factor VII (also: proconvertin, factor VIIa, EC 3.4.21.21), factor IX (also: antihemophilic factor B, Christmas factor, EC 3.4.21.22), factor X (also: Stuart-Prower factor, factor Xa, EC 3.4.21.6), factor XI (plasma thromboplastin antecedent, EC 3.4.21.27), factor XII (Hageman factor, EC 3.4.21.28), prekallikrein (Fletcher factor, EC 3.4.21.34), protein C (EC 3.4.21.69), plasminogen (EC 3.4.21.7, including Lys-plasminogen, Glu-plasminogen, plasmin (including Lys-plasmin, Glu-plasmin)), tissue plasminogen activator (also tPA, PLAT, EC 3.4.21.68), and urokinase (also: urokinase-type plasminogen activator, uPA, EC 3.4.21.73). It will be understood that in principle all isotypes, zymogens and matured forms of the aforementioned enzymes are included in the sense of the present invention. Alternatively, the coagulation factor may also be factor VIII (also: anti-hemophilic factor (AHF)) or factor V (also: proaccelerin, labile factor).

Particularly preferably, the enzymatic entity E is characterized in that it is plasminogen (EC 3.4.21.7, including Lys-plasminogen, Glu-plasminogen, plasmin (including Lys-plasmin, Glu-plasmin)).

As indicated above, either binding moiety A3 or binding moiety A2 may be bound to the solid phase S. It will be understood that the term "bound to the solid phase S" may be understood in the broadest sense as covalently or non-covalently bound to the solid phase S. Such binding may be a direct binding or may be binding to any linker suitable for this purpose except a linker comprising the enzymatic entity E. In this context, a linker may be any linker suitable for this purpose. Exemplarily, a linker between the respective binding moiety A2 or A3 and the solid phase S may comprise biotinyl residue, an organosilanyl residue, a succinimidyl residue, a maleimidyl residue, an aminoacyl recidue, a diamine alkyl residue, a dicarboxy residue, a non-toxic polymer (e.g., polyethylene glycol (PEG)), or a combination of two or more thereof. Preferably, the linker does not comprise more than 50, in particular not more than 20 carbon atoms. The person skilled in the art will be aware of numerous possibilities of conjugations. This binding of A2 or A3 to the solid phase occurs before conducting step (ii) of contacting the liquid L with the solid phase S. Therefore, the binding of A2 or A3 that is characterized as being "bound to the solid phase S" is independent on the presence of E.

In a highly preferred embodiment, the third binding moiety A3 is bound to the solid phase S and the second binding moiety A2 bears M-II, preferably wherein the amount of M-I immobilized on the solid phase S indicates the content of E in L and the amount of M-II immobilized on the solid phase S indicates the content and accessibility of the active sites of E in L.

Accordingly, then the method of the present invention comprises the following steps:
(i) providing the following:
   (A1) a first binding moiety A1 that binds to a first binding site B1 of E that is different from the ligand binding site B2 in the active site of E, wherein A1 bears a first detectable moiety M-I,
   (A2) a second binding moiety A2 that binds to B2, wherein A2 bears a second detectable moiety M-II that is different from M-I, and
   (A3) a third binding moiety A3 that binds to a third binding site B3 of E that is different from B1 and B2, wherein A3 is bound to the solid phase S,
   wherein A1 and A2 are not bound to the solid phase S;
(ii) contacting L comprising E with A1, A2 and A3 and enabling binding of A1, and A3 to E;
(iii) removing unbound A1 and A2 from the solid phase S; and
(iv) determining the amount of M-I and M-II immobilized on the solid phase S.

Upon conducting step (ii) a complex of A1, optionally A2, and A3 each bound to E is formed which is immobilized on the solid phase S via A3. In other words, when the enzymatic entity E bears the active site comprising the binding site B2, the following complex may be formed:
[solid phase S]-A3-E(A1)(A2),
wherein A1 bears the detectable marker M-I and A2 bears the detectable marker M-II and wherein M-I and M-II are found to be immobilized in the solid phase S. When the enzymatic entity E does not bear the active site comprising the binding site B2, the following complex may be formed:
[solid phase S]-A3-E(A1),
wherein A1 bears the detectable marker M-I and A2 bears the detectable marker M-II and wherein only M-I is found to be immobilized in the solid phase S, whereas M-II is not immobilized. When the enzymatic entity E is not present, the following is found:
[solid phase S]-A3,
wherein A1 bears the detectable marker M-I and A2 bears the detectable marker M-II and wherein M-I as well as M-II are not immobilized on the solid phase S. Then, the amount of M-I immobilized on the solid phase S may indicate the content of E in L and the amount of M-II immobilized on the solid phase S may indicate the content and accessibility of the active sites of E in L.

In an alternative preferred embodiment, the second binding moiety A2 is immobilized on the solid phase S, preferably wherein the amount of M-I immobilized on the solid phase S indicates the content and accessibility of the active sites of E in L, and wherein the third binding moiety A3 is preferably absent.

Accordingly, then the method of the present invention comprises the following steps:
(i) providing the following:
   (A1) a first binding moiety A1 that binds to a first binding site B1 of E that is different from the ligand binding site B2 in the active site of E, wherein A1 bears a first detectable moiety M-I, and
   (A2) a second binding moiety A2 that binds to B2, wherein A2 is bound to the solid phase S,
   wherein A1 is not bound to the solid phase S;
(ii) contacting L comprising E with A1 and A2 and enabling binding of A1 and A2 to E;
(iii) removing unbound A1 and A2 from the solid phase S; and
(iv) determining the amount of M-I immobilized on the solid phase S.

Upon conducting step (ii) a complex of A1 and A2 each bound to E is formed which is immobilized on the solid phase S via A2. In other words, when the enzymatic entity E bears the active site comprising the binding site B2, the following complex may be formed:
[solid phase S]-A2-E(A1),
wherein A1 bears the detectable marker M-I and wherein M-I is found to be immobilized in the solid phase S. When the enzymatic entity E does not bear the active site comprising the binding site B2, the following is found:
[solid phase S]-A2, wherein A1 bears the detectable marker M-I and wherein M-I is not immobilized on the solid phase S. Then, the amount of M-I immobilized on the solid phase S may indicate the content and accessibility of the active sites of E in L.

Furthermore, it may be of interest to concomitantly obtain information on the enzymatic isotype. Exemplarily, it may be of interest to know the content of the plasminogen-isotypes Lys-plasminogen, Glu-plasminogen, Lys-plasmin and Glu-plasmin.

Therefore, in a preferred embodiment, the method of the invention is further characterized in that:
step (i) further comprises providing a forth binding moiety A4 that binds to a binding site B4 that is not present in all isotypes of E, wherein B4 is different from each of B1 and B2, and, if A3 is present, B3, wherein A4 bears a further detectable moiety M-III that is different from M-I and, if present, M-II;
step (ii) further comprises binding of A4 to E;
step (ii) further comprises removing unbound A4 from the solid phase S; and step (iv) further comprises determining the amount of M-III, preferably wherein the amount of immobilized M-III indicates the content of the one or more B4-comprising isotypes of E in L.

Accordingly, in a highly preferred embodiment, the method of the present invention comprises the following steps:
(i) providing the following:
   (A1) a first binding moiety A1 that binds to a first binding site B1 of E that is different from the ligand binding site B2 in the active site of E, wherein A1 bears a first detectable moiety M-I,
   (A2) a second binding moiety A2 that binds to B2,
   (A3) optionally a third binding moiety A3 that binds to a third binding site B3 of E that is different from B1 and B2, and
   (A4) a forth binding moiety A4 that binds to a binding site B4 that is not present in all isotypes of E, wherein B4 is different from each of B1 and B2, and, if A3 is present, B3,
   wherein a single one of A3 or A2 is bound to a solid phase S, and wherein, if A3 is present, the other one of A2 or A3 not bound to the solid phase S bears a second detectable moiety M-II that is different from M-I, and wherein A4 bears a further detectable moiety M-III that is different from M-I and, if present, M-II;
(ii) contacting L comprising E with A1, A2 and A4 and, if present, A3 and enabling binding of A1 and A2 and, if present, A3 to E;
(iii) removing unbound A1, A2 and A4 and, if present, A3 from the solid phase S; and
(iv) determining the amount of M-I and M-III and, if present, M-II immobilized on the solid phase S.

In a highly preferred embodiment, the method of the present invention comprises the following steps:
(i) providing the following:
   (A1) a first binding moiety A1 that binds to a first binding site B1 of E that is different from the ligand binding site B2 in the active site of E, wherein A1 bears a first detectable moiety M-I,
   (A2) a second binding moiety A2 that binds to B2, wherein A2 bears a second detectable moiety M-II that is different from M-I,
   (A3) a third binding moiety A3 that binds to a third binding site B3 of E that is different from B1 and B2, wherein A3 is bound to the solid phase S, and
   (A4) a forth binding moiety A4 that binds to a binding site B4 that is not present in all isotypes of E, wherein B4 is different from each of B1, B2 and B3,
   wherein A1, A2 and A3 are each not bound to the solid phase S, and wherein A4 bears a further detectable moiety M-III that is different from M-I and M-II;
(ii) contacting L comprising E with A1, A2, A3 and A4 and enabling binding of A1, A2, A3 and A4 to E;
(iii) removing unbound A1, A2, A3 and A4 from the solid phase S; and
(iv) determining the amount of M-I, M-II and M-III immobilized on the solid phase S.

Then, the amount of immobilized M-III may indicate the content of the one or more B4-comprising isotypes of E in L.

Upon conducting step (ii) a complex of A1, A3, optionally A2, and optionally A4 each bound to E is formed which is immobilized on the solid phase S via A3. In other words, when the enzymatic entity E bears the active site comprising the binding site B2 and concomitantly is of the isotype of interest which is detected by A4, the following complex may be formed:
[solid phase S]-A3-E(A1)(A2)(A4),
wherein A1 bears the detectable marker M-I, A2 bears the detectable marker M-II, A4 bears the detectable marker M-III and wherein M-I, M-II and M-III are found to be immobilized in the solid phase S. When the enzymatic entity E does not bear the active site comprising the binding site B2 but is of the isotype of interest which is detected by A4, the following complex may be formed:
[solid phase S]-A3-E(A1)(A4),

When the enzymatic entity E bears the active site comprising the binding site B2 but is not of the isotype of interest which is detected by A4, the following complex may be formed:
[solid phase S]-A3-E(A1)(A2),

When the enzymatic entity E does not bear the active site comprising the binding site B2 but is not of the isotype of interest which is detected by A4, the following complex may be formed:
[solid phase S]-A3-E(A1),

When the enzymatic entity E is not present, the following is found:
[solid phase S]-A3,

Then, the amount of M-I immobilized on the solid phase S may indicate the content of E in L, the amount of M-II immobilized on the solid phase S may indicate the content and accessibility of the active sites of E in L and the amount of immobilized M-III may indicate the content of the one or more B4-comprising isotypes of E in L.

In an alternative preferred embodiment, the method of the present invention comprises the following steps:
(i) providing the following:
   (A1) a first binding moiety A1 that binds to a first binding site B1 of E that is different from the ligand binding site B2 in the active site of E, wherein A1 bears a first detectable moiety M-I,
   (A2) a second binding moiety A2 that binds to B2, wherein A2 is bound to the solid phase S, and
   (A4) a further binding moiety A4 that binds to a binding site B4 that is not present in all isotypes of E, wherein B4 is different from each of B1 and B2, and wherein A4 bears a further detectable moiety M-III different from M-I,
   wherein A1 and A4 are each not bound to the solid phase S;
(ii) contacting L comprising E with A1, A2 and A4 and enabling binding of A1, A2 and A4 to E;
(iii) removing unbound A1, A2 and A4 from the solid phase S; and
(iv) determining the amount of M-I and M-III immobilized on the solid phase S.

Upon conducting step (ii) a complex of A1 and A2 and optionally A4, each bound to E is formed which is immobilized on the solid phase S via A2. In other words, when the enzymatic entity E bears the active site comprising the binding site B2 and concomitantly is of the isotype of interest which is detected by A4, the following complex may be formed:
[solid phase S]-A2-E(A1)(A4), wherein A1 bears the detectable marker M-I and A4 bears the detectable marker M-III, and wherein M-I and M-III are found to be immobilized in the solid phase S.

When the enzymatic entity E bears the active site comprising the binding site B2 and is not of the isotype of interest which is detected by A4, the following complex may be formed:
[solid phase S]-A2-E(A1).

When the enzymatic entity E does not bear the active site comprising the binding site B2, the following is found:
[solid phase S]-A2,

Then, the amount of M-I immobilized on the solid phase S may indicate the content and accessibility of the active sites of E in L and the amount of immobilized M-III may indicate the content of the one or more B4-comprising isotypes of E in L

In a preferred embodiment, when A4 is also used, the method further comprises the step of comparing the determined content of the one or more isotypes of interest of the enzymatic entity E with
(a) a predetermined reference value indicating the dependency of signal intensity arising from M-III to the content of the one or more isotypes, or
(b) the content of the one or more isotypes with of the signal intensity arising from M-IIII in a control series of various contents of the one or more isotypes in control samples measured under comparable conditions, preferably in the same measurement series.

Each of the binding moieties A1, A2, A3 and A4 may be selected independently from another be any binding moiety that specifically binds to its target.

In a preferred embodiment, at least one of the binding moieties selected from the group consisting of A1 and, if present, A3 and, if present, A4 is an antibody or fragment or analogue thereof.

In a more preferred embodiment, all of A1 and, if present, A3 and, if present, A4 are each independently selected from an antibody or fragment or analogue thereof.

The term "antibody" as used herein may be understood in the broadest sense and also includes what may be designated as an antibody variant (also: antibody mutant). As used in the context of the present invention, the terms "antibody variant" and "antibody mutant" may be understood interchangeably in the broadest sense as any antibody mimetic or antibody with altered sequence known in the art. The antibody variant may have at least 10%, at least 20%, at least 50%, at least 60%, at least 70%, at least 80%, at least 85%, at least 90% or at least 95% of the binding affinity of a corresponding antibody, i.e., bear a dissociation constant (Kd) of less than 10 µM, less than 1 µM, less than 500 nM, less than 200 nM, less than 100 nM, less than 50 nM, less than 40 nM, less than 30 nM or even less than 20 nM. The term "antibody" may also include an antibody mimetic or any (natural) ligand of fragments thereof which may be understood in the broadest sense as organic compounds that, like antibodies, can specifically bind antigens and that typically have a molecular mass in a range of from approximately 3,000 Da to approximately 25,000 Da.

It may be any (natural) ligand or fragments of them. Antibody mimetics may be, e.g., affibody molecules (affibodies), aptamers, affilins, affitins, anticalins, avimers, DARPins, Fynomers, Kunitz domain peptides, single-domain antibodies (e.g., VHH antibodies or VNAR antibodies, nanobodies), monobodies, diabodies, triabodies, flexibodies and tandabs.

The antibody mimetics may be of natural origin, of gene technologic origin and/or of synthetical origin. The antibody mimetics may also include polynucleotide-based binding units. Optionally, the antibody may also be a CovX-body. Optionally, the antibody may also be a cameloid species antibody. The antibody according to the present invention is preferably a monoclonal antibody, a chimeric antibody or a humanized antibody. Such humanized antibodies are also described in the art (cf., exemplarily, pages 175-177 of "Molekulare Biotechnologie - Grundlagen und Anwendungen", 2009, eds. Clark and Pazdernik, Spectrum Akademischer Verlag Heidelberg). Monoclonal antibodies are monospecific antibodies that are identical because they are produced by one type of immune cell that are all clones of a single parent cell. Such monoclonal antibodies are also described in the art (cf., exemplarily, pages 174 and 175 of "Molekulare Biotechnologie - Grundlagen und Anwendungen", 2009, eds. Clark and Pazdernik, Spectrum Akademischer Verlag Heidelberg). A chimeric antibody is an antibody in which at least one region of an immunoglobulin of one species is fused to another region of an immunoglobulin of another species by genetic engineering in order to reduce its immunogenicity. For example murine V_{L} and V_{H} regions may be fused to the remaining part of a human immunoglobulin.

As used herein, the term "antibody fragment" may be understood in the broadest sense as any fragment of an antibody that still bears binding affinity to its molecular target. Exemplarily, the antibody fragment may be a fragment antigen binding (Fab fragment), F(ab')₂, Fab', Fc, scFv, a truncated antibody comprising one or both complementarity determining region(s) (CDR(s)) or the variable fragment (Fv) of an antibody. Variable fragments (Fvs) are the smallest fragments with an intact antigen-binding domain consisting of one V_{L} and one V_{H}. Such fragments, with only the binding domains, can be generated by enzymatic approaches or expression of the relevant gene fragments, e.g. in bacterial and eukaryotic cells. Different approaches can be used, e.g. either the Fv fragment alone or Fab'-fragments comprising one of the upper arms of the "Y" that includes the Fv plus the first constant domains.

These fragments are usually stabilized by introducing a polypeptide link between the two chains which results in the production of a single chain Fv (scFv). Alternatively, disulfide-linked Fv (dsFv) fragments may be used. The binding domains of fragments can be combined with any constant domain in order to produce full length antibodies or can be fused with other polypeptides and polypeptides. A recombinant antibody fragment is the single-chain Fv (scFv) fragment. Dissociation of scFvs results in monomeric scFvs, i.e. mono-Fvs, which can be complexed into dimers (diabodies), trimers (triabodies) or larger aggregates such as TandAbs and Flexibodies. The antibody fragment may be a Fab, a F(ab')₂, a Fab', an (optionally disulfide-linked) Fv, a scFv, a (scFv)₂, a bivalent antibody, a bispecific antibody, a multispecific antibody, a diabody, a triabody, a tetrabody or a minibody. Examples of such antibody fragments are described in standard textbooks in the art (cf., exemplarily, pages 178-181 of "Molekulare Biotechnologie - Grundlagen und Anwendungen", 2009, eds. Clark and Pazdernik, Spectrum Akademischer Verlag Heidelberg).

The antibody or antibody fragment, independent on its chemical nature, may optionally be dissolved in any medium suitable for storing said antibody such as, e.g., water, an aqueous buffer (e.g., a Hepes, Tris, or phosphate buffer (e.g. phosphate buffered saline (PBS)), an organic solvent (e.g., dimethyl sulfoxide (DMSO), dimethylformide (DMF)) or a mixture of two or more thereof. The antibody or variant thereof according to the present invention may be of any species or origin. It may bind to any epitope(s) comprised by its molecular target structure (e.g., linear epitope(s), structural epitope(s), primary epitope(s), secondary epitope(s)). Preferably, the antibody or variant thereof may recognize the naturally folded molecular target structure or a domain or fragment thereof. The antibody or variant thereof may be of any origin an antibody may be obtained from such as, e.g., natural origin, a gene technologic origin and/or a synthetic origin. Optionally, the antibody may also be commercially available. An antibody may further comprise one or more posttranscriptional modification(s) and/or may be conjugated to one or more further structures such as label moieties or cell-penetrating peptides (CPPs). Optionally, the antibody or antibody fragment may be added to a support, particularly a solid phase such as an array, bead (e.g. glass or magnetic), a fiber, a film etc. The skilled person will be able to adapt the antibody of the present invention and a further component to the intended use by choosing a suitable further component.

The detectable units of M-I, M-II and M-III may be each directly or indirectly detectable. As used herein, the term "directly detectable" may be understood in the broadest sense as being detectable without the need of any further molecular compounds. Exemplarily, a chromophore, a fluorophore, a metal label, a radioactive label and a spin label are each directly detectable. As used herein, the term "indirectly detectable" may be understood in the broadest sense as being detectable when at least a further molecular compound that provides a detectable signal is provided. Exemplarily, an enzyme catalyzing the conversion of a substrate to a detectable product, or a binding site C to which a binding moiety, in particular a secondary antibody or fragment or analogue thereof, are each indirectly detectable.

In a preferred embodiment, each of M-I, M-II and M-III is independently selected from the group consisting of
(1) an enzyme catalyzing the conversion of a substrate to a detectable product;
(2) a chromophore;
(3) a fluorophore;
(4) a metal label, in particular a gold bead or a magnetizable metal bead;
(5) a radioactive label;
(6) a nano- or microparticle;
(7) a binding site C to which a binding moiety, in particular a secondary antibody or fragment or analogue thereof bearing one of (1) to (6) can bind to; and
(8) a combination of two or more of (1) to (7).

As indicated above, depending on the detectable moieties M-I, M-II and M-III, as far as present, used, various routes of detection may be employed such as, exemplarily, colorimetry, enzymometry, turbidimetry, nephelometry, (chemo)luminometry, fluorometry (including quenching- and/or FRET- and/or FRAP-based methods and time-resolved fluorometry), a scintillation assay, or combinations of two or more thereof.

In a highly preferred embodiment, at least one of M-I, M-II and M-III, preferably two or all of M-I, M-II and M-III, as far as present, are selected from an enzyme catalyzing the conversion of a substrate to a detectable product. Such enzyme may exemplarily be selected from a peroxidase (e.g, horseradish peroxidase (HPR) or glucose oxidase (e.g, from an *Aspergillus* species)), a phosphatase (e.g., alkaline phosphatase (AP) or glucose 6-phosphatase), a galactosidase (e.g., beta-galactosidase (βGal)), an urease, lysozyme, and a dehydrogenase of a bicarboxylic acid (e.g., malate dehydrogenase). The use of such enzymes for detection is also A number of usable detection methods is well-known in the art and exemplarily summarized in Kiessig (cf., BioTec (1991) 4:30-34) and/or Porstmann and Kiessig (Journal of Immunological Methods (1992) 150:5-21). Exemplarily, the amount of immobilized peroxidase (e.g, horseradish peroxidase (HPR)) may be determined by the reaction of 3,3',5,5'-tetramethylbenzidine (TMB). Exemplarily, the amount of immobilized phosphatase (e.g., alkaline phosphatase (AP)) may be determined by the detection of para-nitrophenol obtained from the hydrolysis of the substrate para-nitrophenylphosphate (pNPP) facilitated by AP.

The amount of immobilized beta-galactosidase (βGal) may exemplarily be determined by the detection of ortho-nitrophenyl obtained from the hydrolysis of the substrate ortho-nitrophenyl-β-galactoside (ONPG) facilitated by βGal. The aforementioned reactions provide chromophores that are directly detectable and easily quantifiable (e.g., by means of photometry). Such enzymatic turnover may provide (semi)quantitative amounts of the binding moiety (A1, A2, A3 or A4) bearing the detectable moiety (M-I, M-II or M-III) that is immobilized on the solid phase S when detection is performed after a given time in the linear range of the catalysis reaction. Therefore, it is preferred that the detection is performed after a given time in the linear range of the catalysis reaction.

A chromophore may preferably be a small-molecule dye or may be a stained particle (e.g., a latex particle). Such a stained particle may have a mean particle diameter (determinable by light scattering or microscopically) in the nano- or micrometer range. Detection may exemplarily be conducted by colorimetry.

A fluorophore may be any fluorophore known in the art. Exemplarily, a fluorophore may be selected from the group consisting of a small-molecule dye moiety (e.g., an Atto dye moiety (e.g., ATTO 390, ATTO 425, ATTO 465, ATTO 488, ATTO 495, ATTO 520, ATTO 532, ATTO 550, ATTO 565, ATTO 590, ATTO 594, ATTO 610, ATTO 611X, ATTO 620, ATTO 633, ATTO 635, ATTO 637, ATTO 647, ATTO 647N, ATTO 655, ATTO 665, ATTO 680, ATTO 700, ATTO 725, ATTO 740), a Cy dye moiety (e.g., Cy3, Cy5, Cy5.5, Cy7), an Alexa dye moiety (e.g., Alexa Fluor 488, Alexa Fluor 546, Alexa Fluor 647, Alexa Fluor 680, Alexa Fluor 750), a VisEn dye moiety (e.g. VivoTag680, VivoTag750), an S dye (e.g., S0387), a DyLight fluorophore moiety (e.g., DyLight 750, DyLight 800), an IRDye moiety (e.g., IRDye 680, IRDye 800), a fluorescein dye moiety (e.g., fluorescein, carboxyfluorescein, fluorescein isothiocyanate (FITC)), a rhodamine dye moiety (e.g., rhodamine, tetramethylrhodamine (TAMRA)), a former HOECHST dye moiety), a fluorescent polypeptide moiety (e.g., cyan fluorescent protein (CFP), green fluorescent protein (GFP) or yellow fluorescent protein (YFP), red fluorescent protein (RFP), mCherry, etc.), a quantum dot moiety or a combination of two or more thereof. The detection of a fluorophore may exemplarily be conducted by fluorometry, Therefore, the fluorescence intensity of different samples may be compared with another.

A metal label may be exemplarily be detected by colorimetry, turbidiemtry or nephelometry. A gold bead may be also considered as colloidal gold. A magnetizable metal bead may also be detected by means of isolating it via a magnet and/or detecting interference with an electromagnet.

A radioactive label may be any radioactive label that is (semi)quantitatively detectable. It may exemplarily be selected from the group consisting of ³H, ¹⁴C, ¹²³I, ¹²⁴I, ¹³¹I, ³²P, ⁹⁹mTc and lanthanides (e.g., ⁶⁴Gd, europium). In this context, a radioactive label may or may not be suitable for scintillation assays.

A binding site C to which a binding moiety, in particular a secondary antibody or fragment or analogue thereof bearing one of (1) to (5) can bind to preferably is an epitope of said secondary antibody or fragment or analogue thereof. An epitope may be any kind of epitope comprised by its molecular target structure (e.g., linear epitope(s), structural epitope(s), primary epitope(s), secondary epitope(s)). Exemplarily, a binding moiety (A1, A2, A3 or A4) comprising a detectable moiety (M-I, M-I or M-III) may be an antibody. Then, the antigen-binding part thereof may specifically bind to the enzymatic entity E, while the constant region thereof (e.g., the Fc fragment) comprises the binding site C to which the secondary antibody or fragment or analogue thereof may bind to.

A nano- or microparticle, even if unstained, may be detectable by means of turbidiemtry or nephelometry. Such particle may be of any material, in particular any material not (significantly) interfering with the method of the present invention, i.e., not binding to E or A1-A4.

It will be understood that the detectable moiety (M-I, M-I or M-III) may be conjugated with the binding moiety (A1, A2, A3 or A4) bay any means. Exemplarily, the detectable moiety may be covalently or noncovalently bound to the binding moiety directly. It may optionally even form a common polypeptide strand. Alternatively, it may also be conjugated via a linker moiety. Exemplarily, a linker between the detectable moiety and the binding moiety may comprise biotinyl residue, an organosilanyl residue, a succinimidyl residue, a maleimidyl residue, an aminoacyl recidue, a diamine alkyl residue, a dicarboxy residue, a non-toxic polymer (e.g., polyethylene glycol (PEG)), or a combination of two or more thereof. Preferably, the linker does not comprise more than 50, in particular not more than 20 carbon atoms. Alternatively or additionally, it may also comprise a polypeptide moiety. A preferred combination could exemplarily be a biotinyl residue combined with a (strept)avidin moiety binding noncovalently with another. In this context, (strept)avidin may optionally form part of a fusion protein with an enzyme which produces a detectable chromogen, fluorophore or chemiluminescence or may bear a directly detectable dye or fluorophore or may serve as an antigen being detectable by an antibody or fragment or variant thereof. The person skilled in the art will be aware of numerous possibilities of conjugations.

The binding moieties may be bound concomitantly to the enzymatic entity E or one or more of the other binding moieties that have previously be bound and detected may be removed from E prior to binding and detecting one or more other binding moiety/moieties.

In a preferred embodiment, in step (ii) at least two of the binding moieties selected from the group consisting of A1 and A2 and, if present, A3, and, if present, A4, concomitantly bind to E. In a more preferred embodiment, in step (ii) all of A1, A2 and A3 and, if present, A4 concomitantly bind to E.

Contacting binding moieties to the enzymatic entity E may be concomitantly or sequentially. In other words, two, three or all of A1 and A2 and, if present, A3, and, if present, A4 may be added to E in separate solutions or may be contacted with E together, thus, in a common solution comprising two, three or all of A1 and A2 and, if present, A3, and, if present, A4.

In a preferred embodiment, in step (ii) the liquid L is contacted with a composition comprising a mixture of all binding moieties selected from the group consisting of A1 and A2 and, if present, A3, and, if present, A4, that are not bound to the solid phase S.

Particularly preferably, this composition comprising A1, A2, A3 and A4, as far as present, is a liquid composition, in particular a solution such as a buffer wherein A1, A2, A3 and A4, as far as present, are dissolved. In other words, in step (ii) the binding moieties selected from the group consisting of A1 and A2 and, if present, A3, and, if present, A4, that are not bound to the solid phase S, are preferably contacted with E concomitantly. Accordingly, in a highly preferred embodiment, the method of the present invention, step (ii) is defined as follows:
contacting L comprising E concomitantly with A1 and A2 and, if present, A3 and enabling binding of A1 and A2 and, if present, A3 and, if present, A4 to E, wherein A1, A2, A3 and A4, as far as present, form part of a single composition contacted with L comprising E.

It will be understood that a single one of A2 and A3 is bound to the solid phase. The other binding moieties preferably form part of a common solution. When A3 is bound to the solid phase, A1 and A2 and, if present, A4 preferably form part of a common solution contacted with the solid phase S bearing immobilized A3 to which E is bound. When A2 is bound to the solid phase, A1 and A4 as far as present may preferably form part of a common solution contacted with the solid phase S bearing immobilized A2 to which E is bound.

Detection of M-I and, if present, M-II, and, if present, M-III may be conducted sequentially or concomitantly. In a preferred embodiment, in step (iv) the determining of the amount of M-I and, if present, M-II, and, if present, M-III is conducted sequentially. In a particularly preferred embodiment, step (iv) is conducted sequentially and comprises one or more washing steps for removing detectable residues from the preceding detection step. This may enable minimizing interference of the detection of a single detectable moiety with the one or two other detectable moiety/moieties.

Accordingly, in a preferred embodiments, step (iii) comprises the following sub-steps in the following order:
(iv-a) determining the amount of a first detectable moiety selected from the group consisting of M-I and, if present, M-II and, if present, M-III immobilized on the solid phase S, optionally subsequently washing the solid phase S; and
(iv-b) determining the amount of a second detectable moiety selected from the group consisting of M-I and, if present, M-II and, if present, M-III immobilized on the solid phase S, wherein said second detectable moiety is unequal to that detected in step (iv-a), optionally subsequently washing the solid phase S; and
(iv-c) optionally determining the amount of a third detectable moiety selected from the group consisting of M-I and, if present, M-II and, if present, M-III immobilized on the solid phase S, wherein said second detectable moiety is unequal to that detected in steps (iv-a) and (iv-b).

Exemplarily, step (iii) may comprise the following sub-steps in the following order:
(iv-a) determining the amount of a M-I on the solid phase S, optionally washing the solid phase S; and
(iv-b) determining the amount of M-II, optionally subsequently washing the solid phase S; and
(iv-c) optionally determining the amount of M-III immobilized on the solid phase S.

Alternatively, step (iii) may comprise the following sub-steps conducted in the following order (from left to right, separated by "->"):
determining the amount of M-I -> determining the amount of M-II;
determining the amount of M-I -> determining the amount of M-III;
determining the amount of M-II -> determining the amount of M-I;
determining the amount of M-III -> determining the amount of M-I;
determining the amount of M-I -> determining the amount of M-II -> determining the amount of M-III;
determining the amount of M-I -> determining the amount of M-III -> determining the amount of M-II;
determining the amount of M-II -> determining the amount of M-I -> determining the amount of M-III;
determining the amount of M-II -> determining the amount of M-III -> determining the amount of M-I;
determining the amount of M-III -> determining the amount of M-I -> determining the amount of M-II; or
determining the amount of M-III -> determining the amount of M-II -> determining the amount of M-I.

It will be understood that also repeated measurements are also possible.

Accordingly, in a highly preferred embodiment, the method of the present invention comprises step (i) as described herein and the following steps (ii)-(iv):
(ii) contacting L comprising E concomitantly with A1 and A2 and, if present, A3 and enabling binding of A1 and A2 and, if present, A3 and, if present, A4 to E,
   preferably wherein A1, A2, A3 and A4, as far as present, form part of a single composition contacted with L comprising E;
(iii) removing unbound A1 and A2 and, if present, A3 and, if present, A4 from the solid phase S; and
(iv-a) determining the amount of a first detectable moiety selected from the group consisting of M-I and, if present, M-II and, if present, M-III immobilized on the solid phase S, optionally washing the solid phase S; and
(iv-b) determining the amount of a second detectable moiety selected from the group consisting of M-I and, if present, M-II and, if present, M-III immobilized on the solid phase S, wherein said second detectable moiety is unequal to that detected in step (iv-a), optionally subsequently washing the solid phase S; and
(iv-c) optionally determining the amount of a third detectable moiety selected from the group consisting of M-I and, if present, M-II and, if present, M-III immobilized on the solid phase S, wherein said second detectable moiety is unequal to that detected in steps (iv-a) and (iv-b).

As can be seen from the above, the present invention also relates to a composition comprising two or more of the unbound binding moieties selected from the group consisting of A1, A2, A3 and A4.

Accordingly, a further aspect of the present invention relates to a composition comprising A1 and A2 not bound to a solid phase S and optionally A4 not bound to a solid phase S, wherein all components are defined as laid out above.

It will be understood that the specifications made in the context of the method as described above apply *mutatis mutandis* to the composition of the present invention.

Accordingly, in a highly preferred embodiment, the method of the present invention comprises the following steps:
(i) providing the following:
   (A1) a first binding moiety A1 which is an antibody or fragment or analogue thereof that binds to a first binding site B1 of E that is different from the ligand binding site B2 in the active site of E, wherein A1 bears a first detectable moiety M-I,
   (A2) a second binding moiety A2 which is an antibody or fragment or analogue thereof that binds to B2,
   (A3) optionally a third binding moiety A3 which is an antibody or fragment or analogue thereof that binds to a third binding site B3 of E that is different from B1 and B2, and
   (A4) optionally a forth binding moiety A4 which is an antibody or fragment or analogue thereof that binds to a binding site B4 that is not present in all isotypes of E, wherein B4 is different from each of B1 and B2, and, if A3 is present, B3,
   wherein a single one of A3 or A2 is bound to a solid phase S, and wherein the other moiety/moieties are not bound to the solid phase S, wherein A3, if present, bears a further detectable moiety M-II and wherein A4, if present, bears a further detectable moiety M-III,
(ii) contacting L comprising E with a composition comprising: A1 and A2 and, if present, A4 and, if present, A3,
   and enabling binding of A1 and A2 and, if present, A4 and, if present, A3 to E,
   preferably wherein A1 and A2 and, if present, A4 and, if present, A3 concomitantly bind to E;
(iii) removing unbound A1 and A2 and, if present, A4 and, if present, A3 from the solid phase S; and
(iv) determining the amount of M-I and, if present, M-II and, if present, M-III immobilized on the solid phase S, wherein said step (iv) is conducted sequentially and comprises one or more washing steps for removing detectable residues from the preceding detection step.

In a particularly preferred embodiment, the method of the present invention comprises the following steps:
(i) providing the following:
   (A1) a first binding moiety A1 which is an antibody or fragment or analogue thereof that binds to a first binding site B1 of E that is different from the ligand binding site B2 in the active site of E, wherein A1 bears a first detectable moiety M-I,
   (A2) a second binding moiety A2 which is a substrate analogue (inhibitor) that binds to B2, wherein A2 bears a second detectable moiety M-II that is different from M-I, and
   (A3) a third binding moiety A3 which is an antibody or fragment or analogue thereof that binds to a third binding site B3 of E that is different from B1 and B2, wherein A3 is bound to the solid phase S, and
   (A4) optionally a forth binding moiety A4 which is an antibody or fragment or analogue thereof that binds to a binding site B4 that is not present in all isotypes of E, wherein B4 is different from each of B1 and B2, and, if A3 is present, B3, wherein A4 bears a second detectable moiety M-III that is different from M-I and M-II,
   wherein A1 and A2 and, if present A4 are not bound to the solid phase S;
(ii) contacting L comprising E with A1, A2 and A3 and, if present, A4 and enabling binding of A1, A2 and A3 and, if present, A4 to E, preferably wherein A1, A2 and A3 and, if present, A4 concomitantly bind to E;
(iii) removing unbound A1 and A2 and, if present, A4 from the solid phase S; and
(iv) determining the amount of M-I and M-II and, if present, M-III immobilized on the solid phase S, wherein said step (iv) is conducted sequentially and comprises one or more washing steps for removing detectable residues from the preceding detection step.

Preferably, the composition comprising A1 and A2 and optionally A4 is a liquid composition, in particular a solution such as a buffer wherein A1 and A2 and optionally A4 are dissolved.

In a particularly preferred example, the method of the present invention in for (concomitantly) determining the content of a (serine) protease (e.g., factor XI (plasma thromboplastin antecedent, EC 3.4.21.27) as component E and the content and accessibility of one or more active site(s) of E in a liquid L, said method comprising the following steps:
(i) providing the following:
   (A1) a first binding moiety A1 which is an antibody that binds to a first binding site B1 of E that is different from the ligand binding site B2 in the active site of E, wherein A1 bears a first detectable moiety M-I, preferably wherein M-I is horseradish peroxidase,
   (A2) a second binding moiety A2 which is a substrate analogue (inhibitor) that binds to B2, wherein A2 bears a second detectable moiety M-II, that is different from M-I, preferably wherein M-II is alkaline phosphatase, and
   (A3) a third binding moiety A3 which is an antibody that binds to a third binding site B3 of E that is different from B1 and B2, wherein A3 is bound to the solid phase S, and
   (A4) optionally a forth binding moiety A4 which is an antibody or fragment or analogue thereof that binds to a binding site B4 that is not present in all isotypes of E, wherein B4 is different from each of B1 and B2, and, if A3 is present, B3, wherein A4 bears a second detectable moiety M-III that is different from M-I and M-II,
   wherein A1 and A2 and, if present A4 are not bound to the solid phase S; (ii-a) contacting L comprising E with A3 bound to the solid phase S and enabling binding of E to this binding moiety bound to the solid phase S, preferably followed by at least one washing step; and
(iii-a) removing unbound A3 from the solid phase S, preferably followed by at least one washing step;
(ii-b) contacting L comprising E (concomitantly or sequentially) with A1 and A2 and, if present, A4 not bound to the solid phase S and enabling binding of E to A1 and A2 and, optionally, A3;
(iii-b) removing unbound A1 and A2 and, if present, A4 from the solid phase S, preferably followed by at least one washing step; and
(iv) determining the amount of M-I and M-II and, if present, M-III immobilized on the solid phase S, wherein said step (iv) is conducted sequentially and comprises one or more washing steps for removing detectable residues from the preceding detection step,
   preferably wherein:
   M-I is horseradish peroxidase and detection of the amount of bound A1 (and thereby M-I) is performed by detecting the turnover of 3,3',5,5'-tetramethylbenzidine (TMB) after a given time in the linear range of the catalysis reaction; and/or
   M-II is alkaline phosphatase and detection of the amount of bound A2 (and thereby M-II) is performed by detecting the amount of para-nitrophenol obtained from the hydrolysis of the substrate para-nitrophenylphosphate (pNPP) after a given time in the linear range of the catalysis reaction

As indicated above, also A2 may be bound to the solid phase S. Accordingly, a further aspect of the present invention relates to a composition comprising A1 and A3 not bound to a solid phase S and optionally A4 not bound to a solid phase S, wherein all components are defined as laid out above.

It will be understood that the specifications made in the context of the method as described above apply *mutatis mutandis* to the composition of the present invention. Preferably, the composition comprising A1 and A3 and optionally A4 is a liquid composition, in particular a solution such as a buffer wherein A1 and A3 and optionally A4 are dissolved.

The components usable for a method of the present invention may also form part of a kit.

Accordingly, a still further aspect of the present invention relates to a kit for conducting the method of the present invention, comprising:
a first binding moiety A1; and
a second binding moiety A2;
optionally a third binding moiety A3; and
optionally a third binding moiety A4; and
optionally user instructions how to conduct the method of the present invention; and
optionally a solid phase S to which either a single one of A2 or A3 is bound or enabling binding to A2 or A3,
wherein all components are defined as laid out above.

It will be understood that the specifications made in the context of the method as described above apply *mutatis mutandis* to the kit of the present invention. The detailed composition of such kit will depend on the details of the method as laid out above. A kit may generally include a package with one or more containers holding the reagents, as one or more separate compositions or, optionally, as a mixture if reagents are compatible. The kit may also include other material(s), which may be desirable from a user standpoint, such as a buffer(s), a diluent(s), a standard(s), and/or any other material useful in sample processing, washing, or conducting any other step of the assay.

In a preferred embodiment, the kit comprises:
a first binding moiety A1 bearing M-I not bound to the solid phase S;
a second binding moiety A2 bearing M-II not bound to the solid phase S; and
a third binding moiety A3 bound to the solid phase S; and
optionally a further binding moiety A4 bearing M-III not bound to the solid phase S; and
optionally user instructions how to conduct the method of the present invention.

In an alternative preferred embodiment, the kit comprises:
a first binding moiety A1 bearing M-I not bound to the solid phase S;
a second binding moiety A2 bound to the solid phase S; and
optionally a further binding moiety A3 bearing M-II not bound to the solid phase S; and
optionally a further binding moiety A4 bearing M-III not bound to the solid phase S; and
optionally user instructions how to conduct the method of the present invention.

A kit according to the present invention may optionally include a solid phase S on which the respective binding moiety A2 or A3 is immobilized. Alternatively, a kit according to the present invention may include a solid phase S and a capture agent affixed to the solid phase S, wherein the capture agent is an antibody specific for the respective binding moiety A2 or A3.

Furthermore, a kit according to the present invention may preferably further comprise user instructions for carrying out the method of the present invention. Instructions included in kits of the invention may be affixed to packaging material or may be included as a package insert. While the instructions are typically written or printed materials they are not limited to such. Any medium capable of storing such instructions and communicating them to an end user is contemplated by this invention. Such media include, for example, computer media including, but are not limited to, electronic storage media (e.g., magnetic discs, tapes, cartridges, chips), optical media (e.g., CD ROM), and the like.

The following Examples as well as the accompanying Figures are intended to provide illustrative embodiments of the present invention described and claimed herein.

### Brief Description of the Figures

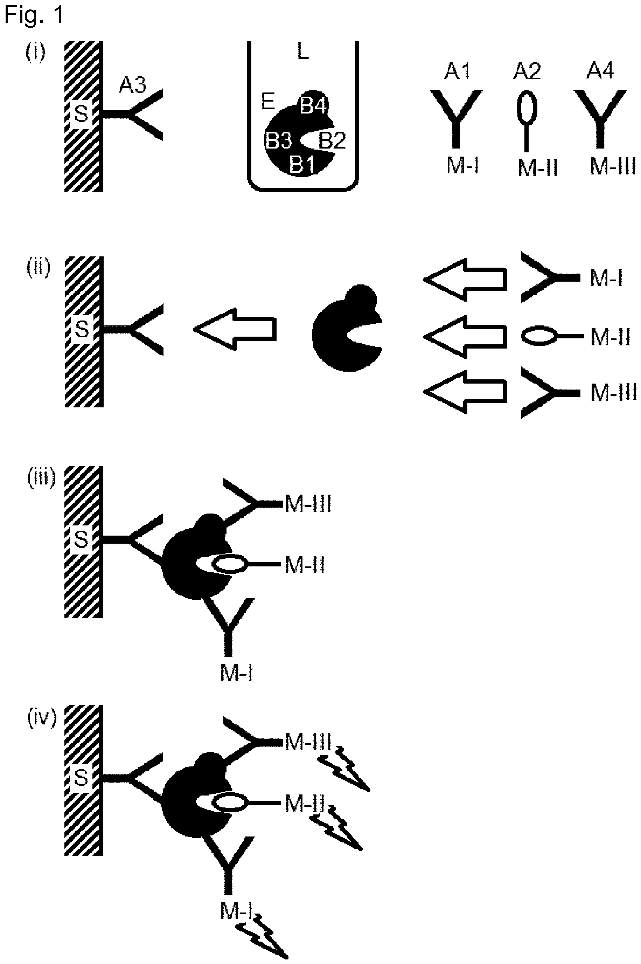

**Figure 1** shows a schematic overview of an embodiment of the present invention. In step (i), the components are provided (from left to right):
   - a solid phase S on which the binding moiety A3 has been immobilized,
   - liquid L comprising an enzymatic entity E that bears binding sites B1-B4,
   - a binding moiety A1 bearing detectable moiety M-I,
   - substrate analogue binding moiety A2 bearing detectable moiety M-II, and
   - an optional binding moiety A4 bearing detectable moiety M-III.
   In step (ii), the components are contacted with another (indicated by arrows).
   The binding moieties each bind to their respective binding sites on E and the unbound binding moieties are removed (step (iii)). Finally, the detectable moieties are detected (indicated by flashes) and their quantity is determined (step (iv)).

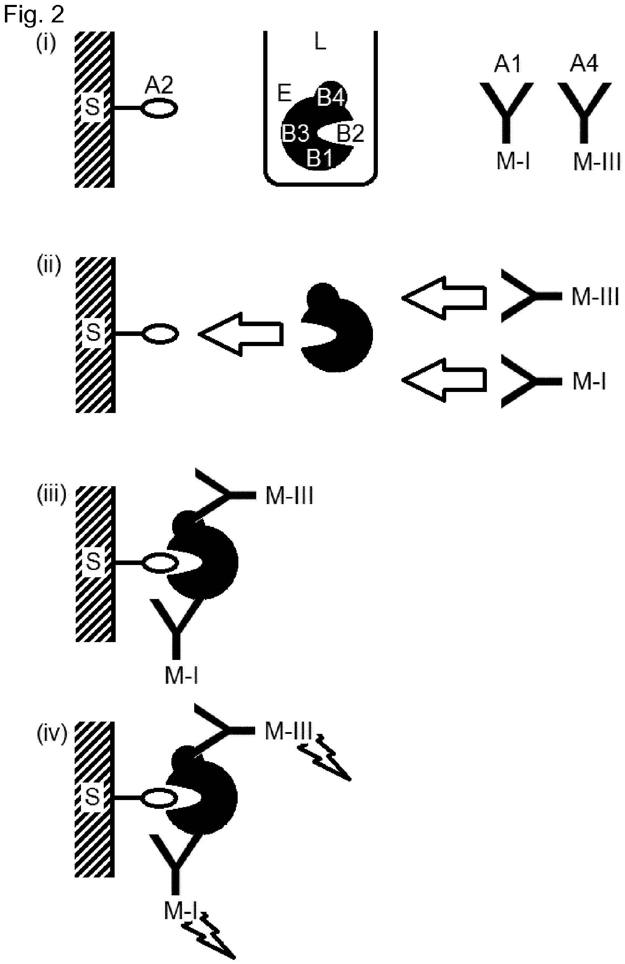

**Figure 2** shows a schematic overview of another embodiment of the present invention. In step (i), the components are provided (from left to right):
   - a solid phase S on which substrate analogue binding moiety A2 has been immobilized,
   - liquid L comprising an enzymatic entity E that bears binding sites B1-B4,
   - a binding moiety A1 bearing detectable moiety M-I, and
   - an optional binding moiety A4 bearing detectable moiety M-III.
   In step (ii), the components are contacted with another (indicated by arrows). The binding moieties each bind to their respective binding sites on E and the unbound binding moieties are removed (step (iii)). Finally, the detectable moieties are detected (indicated by flashes) and their quantity is determined (step (iv)).

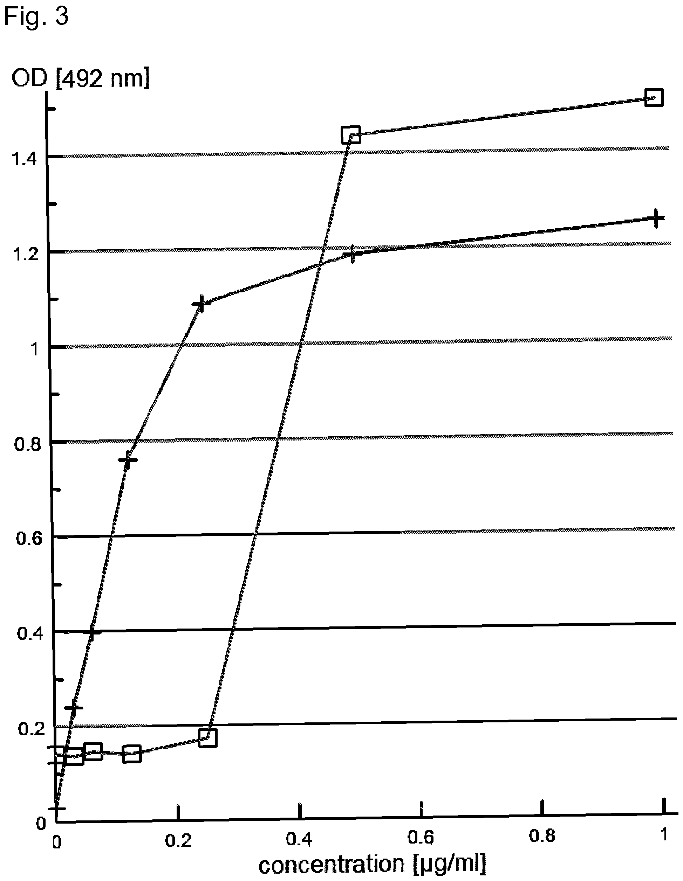

**Figure 3** shows the relationship between variant concentrations of (recombinant) tissue plasminogen activator ((r)tPA) in combination with Lys-plasminogen (Lys-PL) (depicted as boxes (□)) and (r)tPA without Lys-PL (depicted as crosses (+)) and the conversion of 3,3',5,5'-tetramethylbenzidine (TMB) after 5 min detected by measuring the absorbance (optical density, OD) at 492 nm. The reaction conditions (using the monoclonal antibody 3VPA and biotinylated TPACK) are those depicted in the Example below.

### Example

For the detection of tPA (Tissue plasminogen activator), microtiter plates are coated with a murine anti-tPA monoclonal antibody (3VPA) at a concentration of 5 µg/mL in 0.1 mol/l carbonate buffer pH 9.6 overnight at 4 °C. The plates were washed 3 times using PBS 0.1 % v/v Tween 20 (PBST).

tPA-containing samples were incubated at room temperature for 90 min. Samples, standards and controls were diluted 1:10 in PBST 2% Gelafusal (Serumwerk Bernburg AG, Germany), 0.01 mg/L phenol red (PBSTG). After three washing steps the next incubation uses as indicator biotinylated TPACK (tosyl phenylalanyl chloromethyl ketone [CMK: chloromethyl ketone], Bachem, Bubendorf, CH) was used in a concentration of 0.1 µg/mL in PBSTG and incubated for 90 min at room temperature.

After additional 3 washing steps the bound biotinylated CMK-peptide were detected by streptavidin-HRP (HRP: Horse radish peroxidase) (concentration: 1 µg/mL in PBSTG, incubation time 15 min at room temperature).

After three washing steps, the substrate reaction using TMB was carried out. This reaction was stopped using 1 mol/L H₂SO₄ after 5 min and the absorbance was measured at 492 nm. (Figure 3)

## Claims

1. A method for determining the content of an enzymatic entity E and the content and accessibility of one or more active sites of entity E in a liquid L, said method comprising the following steps:
(i) providing the following:
(A1) a first binding moiety A1 that binds to a first binding site B1 of E that is different from the ligand binding site B2 in the active site of E, wherein A1 bears a first detectable moiety M-I,
(A2) a second binding moiety A2 that binds to B2, and
(A3) optionally a third binding moiety A3 that binds to a third binding site B3 of E that is different from B1 and B2,
wherein a single one of A3 or A2 is bound to a solid phase S, and wherein, if A3 is present, the other one of A2 or A3 not bound to the solid phase S bears a second detectable moiety M-II that is different from M-I;
(ii) contacting L comprising E with A1 and A2 and, if present, A3 and enabling binding of A1 and A2 and, if present, A3 to E;
(iii) removing unbound A1 and A2 and, if present, A3 from the solid phase S; and
(iv) determining the amount of M-I and, if present, M-II immobilized on the solid phase S.

2. The method of claim 1, wherein A2 is a substrate analogue of E, in particular a substrate analogue inhibitor of E.

3. The method of any of claims 1 or 2, wherein L is obtained from a biological source, preferably wherein L is a body fluid or a liquid derived therefrom, in particular wherein L is blood plasm or a fraction thereof.

4. The method of any of claims 1 to 3, wherein E is **characterized in that** it is or comprises a coagulation factor.

5. The method of any of claims 1 to 4, wherein E is **characterized in that** it is or comprises a protease, in particular a serine protease.

6. The method of any of claims 1 to 5, wherein in step (i) A3 is bound to the solid phase S and A2 bears M-II, preferably wherein the amount of M-I immobilized on the solid phase S indicates the content of E in L and the amount of M-II immobilized on the solid phase S indicates the content and accessibility of the active sites of E in L.

7. The method of any of claims 1 to 5, wherein in step (i) A2 is immobilized on the solid phase S, preferably wherein the amount of M-I immobilized on the solid phase S indicates the content and accessibility of the active sites of E in L, and wherein A3 is preferably absent.

8. The method of any of claims 1 to 7, wherein:
step (i) further comprises providing a forth binding moiety A4 that binds to a binding site B4 that is not present in all isotypes of E, wherein B4 is different from each of B1 and B2, and, if A3 is present, B3, wherein A4 bears a further detectable moiety M-III that is different from M-I and, if present, M-II;
step (ii) further comprises binding of A4 to E;
step (iii) further comprises removing unbound A4 from the solid phase S;
and
step (iv) further comprises determining the amount of M-III, preferably wherein the amount of immobilized M-III indicates the content of the one or more B4-comprising isotypes of E in L.

9. The method of any of claims 1 to 8, wherein at least one of the binding moieties selected from the group consisting of A1 and, if present, A3 and, if present, A4 is an antibody or fragment or analogue thereof; preferably wherein all of A1 and, if present, A3 and, if present, A4 are each independently selected from an antibody or fragment or analogue thereof.

10. The method of any of claims 1 to 9, wherein in step (ii) at least two of the binding moieties selected from the group consisting of A1 and A2 and, if present, A3, and, if present, A4, concomitantly bind to E, preferably wherein in step (ii) all of A1 and A2 and, if present, A3 and, if present, A4 concomitantly bind to E.

11. The method of any of claims 1 to 10, wherein in step (ii) L is contacted with a composition comprising a mixture of all binding moieties selected from the group consisting of A1 and A2 and, if present, A3, and, if present, A4, that are not bound to the solid phase S.

12. The method of any of claims 1 to 11, wherein in step (iv) the determining of the amount of M-I and, if present, M-II, and, if present, M-III is conducted sequentially or concomitantly, preferably wherein step (iv) is conducted sequentially, in particular wherein step (iv) is conducted sequentially and comprises one or more washing steps for removing detectable residues from the preceding detection step.

13. The method of any of claims 1 to 12, wherein each of M-I, M-II and M-III is independently selected from the group consisting of:
(1) an enzyme catalyzing the conversion of a substrate to a detectable product;
(2) a chromophore;
(3) a fluorophore;
(4) a metal label, in particular a gold bead or a magnetizable metal bead;
(5) a radioactive label;
(6) a nano- or microparticle;
(7) a binding site C to which a binding moiety, in particular a secondary antibody or fragment or analogue thereof bearing one of (1) to (6) can bind to; and
(8) a combination of two or more of (1) to (7).

14. A composition comprising:
A1 and A2 not bound to a solid phase S and optionally A4 not bound to a solid phase S; or
A1 and A3 not bound to a solid phase S and optionally A4 not bound to a solid phase S,
wherein all components are defined as laid out in any of the claims 1 to 13.

15. A kit for conducting the method of any of claims 1 to 13, comprising:
a first binding moiety A1; and
a second binding moiety A2;
optionally a third binding moiety A3; and
optionally a third binding moiety A4; and
optionally user instructions how to conduct the method of any of claims 1 to 13; and
optionally a solid phase S to which either a single one of A2 or A3 is bound or enabling binding to A2 or A3,
wherein all components are defined as laid out in any of the claims 1 to 13.
